# EUROPEAN PATENT APPLICATION

(11) **EP 4 411 348 A1**
(43) Date of publication of application: **07.08.2024**
(21) Application number: 23154460.2
(22) Date of filing: 01.02.2023
(51) Int. Cl.: G01N 21/65, G01N 21/64, C12Q 1/02

(54) **A METHOD FOR IDENTIFYING A METABOLIC STATE OF A MICROBE**

(71) Applicant: Mibic GmbH & Co KG, 12459 Berlin (DE)
(72) Inventor: VALET, Oliver, 12459 Berlin (DE); LANKERS, Markus, 12459 Berlin (DE)
(74) Representative: Schulz Junghans Patentanwälte PartGmbB

(57) **Abstract**

The invention relates to a method for identifying a metabolic state of microbes based on a Raman-spectroscopic signal, the method comprising the steps of:
a. Acquiring a sample comprising the microbes,
b. Exposing the sample to a solution comprising a Raman-active compound comprising a Raman-active isotope, wherein said compound is ingestible by the microbes by way of their metabolism,
c. Filtering the sample onto a filter that is designed and configured to retain the microbes on a surface of the filter and to clear the sample from the solution,
d. Imaging the filter surface with the microbes by means of an optical imaging method so as to obtain from the detected signal of the filter surface locations on the filter at which at least microbic residues or microbes are located,
e. On the filter, at least at one obtained location, performing a Raman-spectroscopic measurement configured to be sensitive to the Raman-active isotope and recording a Raman spectrum at the at least one obtained location,
f. From the recorded Raman spectrum recorded at the obtained location, differentiating between at least two metabolic states of the microbe at the time of exposure of the sample to the Raman active isotope by means of analysis of the Raman spectrum with regard to a corresponding signal in the Raman spectrum.

## Description

The invention relates to a method for identifying a metabolic state of microbes based on a Raman-spectroscopic signal in combination with an optical imaging method. The invention further relates to a kit with components for preparing a sample for identifying the metabolic state of a microbe.

Bacterial viability monitoring is an important task in many areas of microbiological research and quality control, including food safety and public health surveillance. Current standard assessments rely largely on the agar plate count method. Viable bacteria in the sample are counted by counting colony forming units (CFU) after an incubation period, assuming that each CFU has grown from one bacterium in the sample. Due to the need for incubation, the process of agar plate counting takes one to five days. In addition, only those cells that can form colonies under the conditions of the experiment are counted, so there is no evidence of dead bacteria or viable but non-culturable (VBNC) cells.

In the art, various methods have been proposed to solve this problem. Some of the methods include the use of Raman-spectroscopic methods that allow to obtain a Raman-spectrum of the sample, and by way of fingerprinting associated the Raman-spectrum to a specific microbe. However, as Raman-spectroscopic recording is typically executed on small volumes, e.g. picoliter volumes, analyzing larger volumes of samples is a lengthy if not impossible task.

Fluorescence-based microplate readers and flow cytometry (FCM) have been employed for the task for counting microbes. However, the accuracy of microplate reader and FCM measurements depends on the sensitivity of the instrument and the quality of the optics, which increases the costs of such systems. In addition, the instruments are bulky and often require operation by trained technicians.

An object of the present invention is to provide a method for identifying a metabolic state of a microbe based on a Raman-spectroscopic signal. The object is achieved by the method having the features of claim 1.

Advantageous embodiments are described in the dependent claims.

According to claim 1, a method for identifying a metabolic state of a microbe based on a Raman-spectroscopic signal is disclosed, wherein the method comprises at least the steps of:
a. Acquiring a sample comprising a plurality of microbes, particularly prokaryotes,
b. Exposing, e.g. by incubating, dissolving, and/or mixing, the sample to a solution comprising a Raman-active compound, such as deuterated water, e.g. in form of D₂O or DHO, with a Raman-active isotope, such as 2H-, 15N- or 13C-comprising compound, that is ingestible by the microbes by way of their metabolism in case the microbes are viable,
c. Filtering the sample onto a filter that is designed and configured to retain the microbes on a surface of the filter and to clear and essentially rid the sample from the solution, and particularly the Raman-active compound that has not been ingested by the microbes,
d. Imaging the filter surface with the labeled microbes by means of an optical imaging method so as to obtain from the detected imaging signal, locations on the filter at which at least microbic residues or one or more microbes is located,
e. On the filter, at one or more obtained locations from the optical imaging method, performing a Raman-spectroscopic measurement configured to be sensitive to the Raman-active isotope, e.g. Deuterium 2H, 15N, and/or 13C, and recording a Raman spectrum at the one or more obtained locations,
f. From the recorded Raman spectrum recorded at the one or more obtained locations, distinguishing between at least two metabolic states of the microbic residue or the one or more microbes at the time of exposure of the sample to the Raman-active compound by means of analysis of the Raman spectrum with regard to a corresponding signal in the Raman spectrum.

The method according to the invention allows for detecting a presence of microbes in a sample, and further to differentiate any detected microbes in terms of their metabolic state in the sample before the microbes have been concentrated on to the filter at the time of incubation with the Raman-active compound.

As the sample is treated with the Raman-active compound that particularly does not interfere with the metabolism of the microbes, ingestion of the compound indicates a viable microbe, wherein dead microbes would not ingest the compound do to a lack of metabolism.

The Raman-active compound may comprise or consist of a Raman-active isotope such as deuterium (2H), 15N or 13C, wherein the numbers "15" in 15N and "13" in 13C refer to the nucleon number of the isotope.

The term "Raman-active" particularly refers to isotopes, such as 2H, 15N, 12C that provide a shift in the Raman peak(s) in a Raman-spectrum in comparison to their nonisotopic counterparts 1H, 14N, 12C that are already ubiquitous in the microbes.

The term "metabolic state" particularly refers to different metabolic states of the microbes. A metabolic state in the meaning of the specification may be one of: viable, non-viable, alive, active, dead or inactive.

The distinction between the two metabolic states of the microbes is made based on the Raman-signal obtained from the sample at the obtained location. Isotope "labelling" by means of the Raman-active compound is particularly cost efficient and accurate.

The isotope labelling of the microbes may not obtained by a specific chemical reaction between the Raman-active compound and the microbe, but by the ingestion of the compound and a subsequent metabolic process which incorporates the isotope of the Raman-active compound into a microbe's component.

Therefore, the step of exposing the sample to the Raman-active compound allows for a generic labelling of viable microbes, independent of their specific nature, kind or species, particularly independent of gram-positive or gram-negative.

In contrast to a viability labelling with a fluorescence marker, the incubation with a Raman-active compound is less expensive and provides a generic as well as reliable labelling approach for all kinds of microbes, particularly in case the Raman-active compound comprises deuterated water.

The filtering step is performed with a filter that is configured and designed for filtering a liquid, particularly aqueous solutions and to retain any particles that exceed a certain size and stiffness.

In order to guide the Raman-measurement, the surface of the filter is imaged for obtaining locations of potential microbes and microbe residues by means of an optical imaging method. This step allows reducing the number of subsequent Raman-measurements, as only at locations on the filter surface where potentially a microbe is detected, a Raman-measurement is executed. This way also the amount of generated data is reduced.

The term "residue" in the context of microbes particularly refers to fragments and/or metabolites of the microbe.

The term "optical imaging" particularly refers to an optical microscopy method that is sensitive enough and has an optical resolution that allows for obtaining locations comprising one or more microbes can be detected.

As the sample is subsequently examined on the surface of same filter by means of a Raman-spectroscopic method at least one location obtained from the optical imaging method, the filter also serves as a sample holder.

After the filtering step, the microbes are immobile on the filter surface which allows Raman-spectroscopic measurements and optical imaging to be executed without any time limit, e.g. due to a flow, in which the microbes may be moving. Also, in comparison to flow-based measurements the challenge to precisely illuminate the volume through which the microbe may flow with the Raman beam is difficult. This problem is solved by the method according to the invention.

Particularly, the filter is formed and configured as a sample holder, particularly wherein the filter comprises a smooth surface on which the microbes are retained and arranged for recording the Raman-signal. The filter surface is particularly configured for optical microscopy imaging and spectroscopic recording of the surface. Therefore, the filter structurally differs from filters configured to filter air volumes for microbes.

The term "corresponding signal in the Raman spectrum" particularly relates to a portion or the whole Raman spectrum that is expected to exhibit a shift and/or a broadening due to the Raman-active compound.

Particularly, the shift and/or broadening of the corresponding signal in the Raman spectrum may be classified during the analysis in at least two classes, each class corresponding to one metabolic state of the at least two metabolic states.

For this purpose, the metabolic states may be predefined states, particularly predefined metabolic states of the microbe such as viable and non-viable.

According to another embodiment, from the recorded Raman spectrum recorded at the obtained location, it is differentiated between exactly two metabolic states of the microbe at the time of exposure of the sample to the Raman active isotope by means of analysis of the Raman spectrum with regard to a corresponding signal in the Raman spectrum.

The term "ingestible" particularly relates to property of the Raman-active compound that renders the compound compatible to and particularly designed for the metabolic uptake of carbon and energy sources to maintain the metabolism. Such a Raman-active compound may comprise 13C labelled glucose.

According to another embodiment of the invention, the microscopy method comprises a label-free optical microscopy method, particularly a dark-field and/or a differential interference contrast (DIC) microscopy method.

The use of a label-free optical microscope imaging method for determining potential locations of microbes on the filter surface allows for a comparably low-cost detection of such locations. Further, the method may be executed faster, as no additional labelling and/or washing step is necessary.

Dark-field as well as DIC microscopy provide high-contrast and selective detection of microbes in comparison to conventional bright field optical imaging methods which may not allow the detection of microbes on the surface due to a too low image contrast.

Prior to the recording of the Raman signal, the filtered sample may be labelled with a luminescent marker. This allows identifying and distinguishing debris, such as inorganic particles, from microbes or microbe residues. This is variant of the invention is illustrated by the following embodiment:

According to another embodiment, additionally or alternatively, a labelling step is executed after the filtering step, wherein the labelling step comprises labelling the filtered sample on the surface with a Raman-spectroscopy-compatible luminescent marker that is configured to label microbes independent of their metabolic state, and wherein the optical imaging method comprises a fluorescence imaging method, particularly as fluorescence microscopy imaging method, configured to detect the luminescence of the luminescent marker.

Luminescence imaging allows for high signal to noise ratios and provides additional selectivity with regard to distinguishing microbes or microbic residues from other kinds of debris on the filter surface, which may not be straightforwardly possible using a label-free optical microscope method.

Prior to optical imaging, a washing step may be executed, wherein the washing step comprises washing the labelled sample so as to clear the sample from unbound luminescent marker.

The term "luminescent" particularly comprises the notion of fluorescent and phosphorescent, as well as varying luminescence-generating mechanisms, such as chemo or electro-luminescence or spontaneous luminescence emission after excitation. The luminescent marker is designed to label microbes or their residues such that a distinction may be made from debris stemming from microbes, and debris that is not of microbial origin, such as inorganic particles or eucaryotes.

Further, the term "Raman-compatible" in connection with the luminescent marker particularly refers to a marker that shows no excitation by a Raman wavelength used for recording the Raman spectrum or a portion of the spectrum, and further exhibits no emission at the probing wavelength of the Raman laser. Alternatively, an interfering luminescent signal may be bleached by the Raman excitation source prior to the acquisition of the Raman-spectrum.

According to another embodiment of the invention, the luminescent marker is a DNA-binding luminescent marker or an intercalating luminescent dye.

This kind of marker allows for a good distinction between microbes and other residues, such as inorganic residues or organic residues that may not be related to the microbe. According to another embodiment of the invention, the luminescent marker is attached to a microbe-specific marker, such as an antibody and/or an antibiotic compound, such as vancomycin (CAS-Nr: 1404-90-6) or polymyxine (e.g. CAS-Nr: 1066-17-7; 1264-72-8), such that the luminescent marker is configured to only bind to gram-positive or only to gram-negative microbes.

This allows for specific labelling of microbes and labelling and selective labelling of specific microbes, which increases accuracy with regard to detection and selectivity of microbes in the sample.

According to another embodiment of the invention, prior to filtering the sample, the sample is treated with a denaturing agent, such as a denaturing detergent, particularly with sodium dodecyl sulfate (SDS, CAS-Nr: 151-21-3) or sodium lauryl sulfate (SLS), particularly rendering the microbes non-viable and/or dead after exposure to the denaturing agent.

As the metabolic state of the microbes has been established by the Raman-active compound prior to filtering, the denaturing allows for labelling with a large variety of luminescent compounds that may not be configured for ingestion/uptake by the microbe.

More importantly, filtering of protein containing samples is difficult or even impossible, as without the denaturing agent the filter would clog. When denaturing the sample after incubation with the Raman-active compound, clogging is prevented, while the microbes are still retained on the filter surface. A drawback of this embodiment is that viability-indicating fluorescence markers for the microbes will not work after denaturing the sample. Therefore, the incubation with a Raman-active compound for viability detection provides an elegant solution to this problem and allows the method to be applied to samples that could not otherwise be processed and analyzed.

According to another embodiment of the invention, the Raman-active compound is deuterated water.

Deuterated water is compatible, non-toxic and basically indistinguishable from water for the microbes and the sample, while exhibiting Raman-active properties, i.e. it allows a distinction in the Raman-spectrum between hydrogen and deuterium and thus allows a distinction regarding the metabolic state of the microbe at the time of incubation with the Raman-active compound at least with respect of whether the compound has been ingested or not by the microbe.

Further, deuterated water is non-luminescent and thus any interference with the luminescent marker is avoided.

According to another embodiment of the invention, a first metabolic state of the at least two metabolic states comprises is an active metabolic state characterized by incorporation of the Raman-active isotope, e.g. Deuterium into metabolic products, such as proteins, RNA, DNA, lipids and/or organelles of the microbe by the microbe, wherein a second metabolic state of the at least two metabolic states comprises or is a dead or inactive state characterized in that after the exposure step the microbe is devoid of the Raman-active isotope e.g. Deuterium incorporated into metabolic products of the microbe.

This embodiment allows distinguishing between microbes in a sample that have been dead or inactive at the time of incubation with the Raman-active isotope and microbes that have been active, e.g. viable at that time.

According to another embodiment of the invention, the surface of the filter is a metallic and reflective surface.

Such a surface provides enhanced Raman-spectroscopic properties, while manufacturing a flat and smooth surface is comparably easy to achieve at the same time.

The filter and thus the filter surface may comprise through holes, through which the liquid may pass during the filtration step. The through holes are so small that microbes cannot pass through, but only liquid of the solution and smaller compounds. According to another embodiment of the invention, the density of the through holes is selected such that the holes, i.e. the aera of the holes covers less than 50%, particularly less than 30% of the filter surface.

According to another embodiment of the invention, the surface of the filter is made of aluminium arranged on top of a polymer layer or wherein the surface is a metal foil, wherein the surface has a thickness between 200 nm to 400 nm, particularly between 220 nm and 250 nm.

According to another embodiment of the invention, the Raman spectroscopic measurement is recorded in the range of 2,100 cm⁻¹ to 2,400 cm⁻¹. This range is particularly sensitive to the shifts of specific Raman-peaks of the Deuterium. This range particularly comprises the C-D vibration of Hydrogen respective Deuterium. According to another embodiment of the invention, from the Raman-spectrum a fraction of microbes in the first state and microbes in the second state is determined, and wherein in case the faction is above or below a predefined threshold value, the method issues an alarm notice.

This embodiment allows to use the method for quality control applications, where the fraction of viable microbes is a value of interest.

The threshold value may be greater than 0.3, particularly wherein the first metabolic state corresponds to an active or viable metabolic state characterized by incorporation of the Raman-isotope and the second metabolic state is a dead or inactive state, characterized by a non-incorporation of the Raman-active isotope.

According to another embodiment of the invention, from the Raman-spectrum a number of microbes in the first state and/or a number of microbes in the second state is determined, and wherein in case one or both numbers are above a predefined threshold value, the method issues an alarm notice.

This embodiment allows to use the method for quality control applications, where the number of viable microbes is a value of interest.

The threshold value may be set to a preselected, e.g. by a customer, number of specific microbes, particularly wherein the first metabolic state corresponds to an active / viable metabolic state characterized by incorporation of the Raman-isotope and the second metabolic state is a dead or inactive state, characterized by a non-incorporation of the Raman-active isotope.

According to another embodiment of the invention, a determination of the metabolic state of the microbe is based on an analysis of only a selected spectral region of the Raman spectrum, particularly a spectral region that comprises information about a presence of the Raman-active isotope in the microbe.

For example, the selected spectral region may comprise the wavenumbers from 2,040 cm⁻¹ to 2,400 cm⁻¹, particularly comprising the C-D-stretching vibrational peak, i.e. caused by the carbon-deuterium bonds, at 2,160 cm⁻¹.

In case for example 13C glucose is used as the Raman-active compound, the selected spectral region may comprise the wavenumbers from 950 cm⁻¹ to 1,010 cm⁻¹, in which the shift of the aromatic ring-vibrations is comprised.

According to another embodiment of the invention, from the recorded Raman spectrum or the selected spectral region, a kind or a type of the microbe is determined, particularly by means of a computer-based and computer-executed comparison to a database of Raman spectra exhibited by different types or kinds of microbes.

This embodiment allows - on top of the determination of whether microbes are present in the sample - a more detailed information on the microbe.

The determination of the kind or type of microbe may include the determination of the microbe species or the microbe itself.

The determination may be facilitated by a pattern recognition or classification method comparing Raman spectra stored in the database with the recorded Raman spectrum. The classification method may comprise a trained neural network or a plurality of trained neural networks trained to detect and classify selected microbes from a Raman-spectrum. Alternatively, or additionally, support vector machines or linear discriminant analysis method may be used to identify the microbe species based on a chemometric characteristic determined derived from the Raman signal recorded at the one or more location.

According to another embodiment of the invention, the sample is selected from the group consisting of meat, fruit, vegetable, body fluid, cell lysate, blood and/or vegetable juice.

The sample may be diluted and/or pre-processed in order to obtain a liquify the sample or render the sample configured for the method according to the invention. Particularly, the sample is an organic sample, food, urine, or the like.

For these samples, the method provides significant advantages as the method is comparably fast, sensitive to minute microbe concentrations and robust.

According to another embodiment of the invention, the sample is exposed to the solution comprising the Raman-active compound, e.g. deuterated water, for less than 8h, particularly less than 2h.

The concentration of deuterated water amounts for example to 50% to 80%, which is sufficient to evoke a sufficiently high Raman-signal in the microbes.

As stated previously, the method therefore allows a comparably fast determination of the presence of microbes, particularly as no amplification or culturing step for the microbes is needed as for example in methods employing an agar-plate.

According to another embodiment of the invention, the method is devoid of a culturing step designed for amplifying and/or multiplying the number of microbes in the sample or in the solution.

This embodiment illustrates the comparably short execution time, which in many industries, such as food, or medical diagnostic industries is an important performance indicator.

According to another embodiment of the invention, the method is executed in less than 4h, particularly, less than 6 hours, particularly wherein the determination of the at least two metabolic state and/or the determination of the fraction of microbes in the first and the second state is takes less than 6 hours counted from and including the acquisition or incubation of the sample.

According to second aspect of the invention, a kit for preparation of a sample for combined Raman spectroscopy and optical, particularly fluorescence imaging is disclosed, wherein the kit comprises components for preparing a sample for identification of a metabolic state of microbes with the method according to the invention, wherein the kit comprises a solution with a Raman-active compound, particularly deuterated water, a luminescent dye configured to label microbes independently of a metabolic state, a filter with through holes, wherein the filter is configured to retain microbes from an aqueous solution on a surface of the filter, wherein the surface of the filter is a metallic and reflective surface, such as to exhibit a diminished Raman-background signal. Further, the filter may comprise any of the features disclosed in the current specification, e.g. in the context of the method according to the invention.

According to another embodiment of the second aspect of the invention, the surface of the filter is made of aluminium arranged on top of a polymer layer or wherein the surface is a metal foil, wherein the surface has a thickness between 200 nm to 400 nm, particularly between 210 nm and 250 nm.

This embodiment provides an enhanced performance and increased signal to noise ratio due to the properties of aluminium or the thin metal foil forming the surface. The properties of aluminium are for example a comparably high reflectivity in the visible region together with a comparably high heat conductivity, which allows using laser intensities that are higher than on other metals.

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.

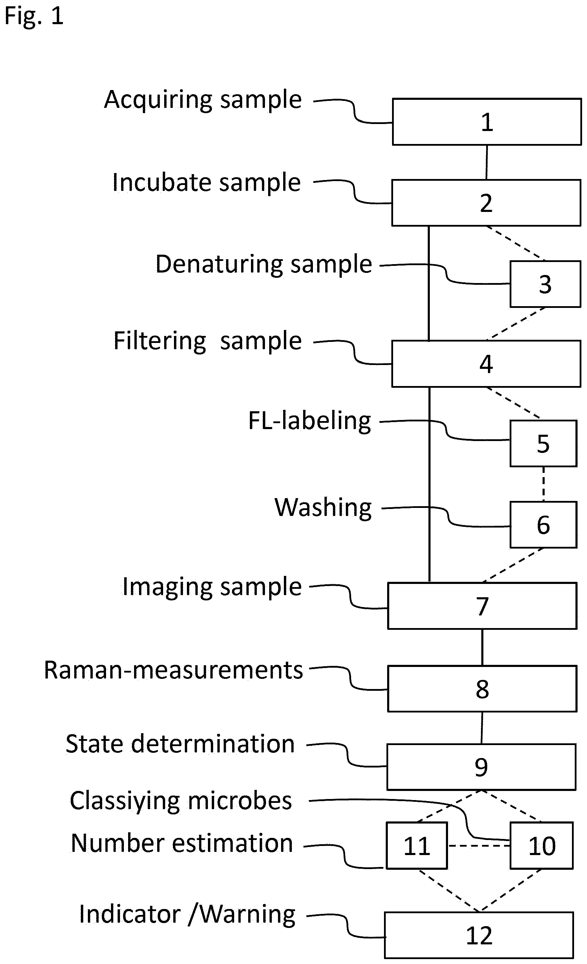

Fig. 1 shows a work-flow diagram of the method according to an exemplary embodiment of the invention.

A workflow according to one embodiment of the invention is schematically depicted in Fig. 1.

In a first step 1, the sample is acquired. Acquisition particularly comprises the liquefaction or liquidation of a solid sample, e.g. comprising blending of the sample, such that the sample may be incubated with the Raman-active compound comprising the Raman-active isotope in a second step 2. Particularly, the isotopes are nonradioactive, i.e. stable isotopes, having *inter alia* the advantage of avoiding any health hazards for the person executing the method.

The Raman-active compound may be deuterated water, such as D₂O, or DHO, wherein the Raman-active isotope is Deuterium (D). Other compounds may be suitable as well, particularly compounds that the microbe may use as a carbon source. The Raman-active compound may also comprise different Raman-active isotopes, such as 2H and 13C.

Incubation time depends on the sample and the Raman-active compound and may range from several minutes up to 6 hours or more. During incubation, the viable and active microbes take up the, e.g. ingest, the Raman active compound or at least the Raman-active isotope. After incubation, the Raman-active isotope is incorporated in living and active microbes, wherein dead or inactive microbes would not have taken up the isotope. At this stage, a marker is obtained, that allows for distinguishing the metabolic state of the microbes based on the incorporation of the isotope in the microbe's components. The incubated sample allows for unambiguous detection of metabolic activity of the microbes in the sample.

In an optional method step 3, the incubated sample may be subjected to a denaturing process, using a denaturing agent, such as SDS, which allows a subsequent clog-free filtering of the sample through a filter that is configured to retain any microbes present in the sample. This step is particularly advantageous in case the sample contains a lot of protein, such as meat. While the metabolites comprising the Raman-active isotope form an integral part of the cell, i.e. the microbe, and, unlike commonly used fluorescent markers, cannot easily leave the cell, various other preparation and preprocessing methods such as the denaturing process may be applied to the sample. It is thereby irrelevant of whether the preparation or preprocessing method leads to the death of the microbe, as is the case in the denaturing process. The isotope label indicative of the metabolic state will remain unaffected from such a treatment.

In a subsequent filtering step 4, the sample is subjected to filtering, during which the microbes are retained on a filter surface of a filter, while other components of the solution that are smaller than the microbes or a less stiff are not retained.

This filtering step 4 reduces on the one hand the volume in which the microbes have been kept and at the same time plates and concentrates the microbes on the filter surface for analysis by further optical methods.

As the filtered and retained sample on the filter surface typically comprises debris from the sample that is not of microbic origin, it is important to identify locations at which microbes or microbe residues are located. For this purpose, in an imaging step 7, an optical imaging method is used for identifying locations that contain microbes on the filter surfaces.

This step allows to subsequently perform targeted Raman-spectroscopic measurements at the identified locations that supposedly contain microbes or microbe residues.

There are several kinds of optical imaging methods. A first optical imaging method comprises a label-free optical microscopy method, such as DIC microscopy or dark-field microscopy.

These methods can be executed comparably cost-efficient and fast, on cost-efficient systems, while providing a sufficiently high image contrast for identifying microbes on the filter surface. Also, no labelling steps is necessary for label-free imaging methods.

From the recorded images, the location(s) of microbes may be obtained, e.g. by way of marking the locations in the image automatically or manually.

Alternatively, or additionally, the optical imaging method may comprise a fluorescence microscopy imaging method.

For this purpose, the microbes may be labelled with a luminescent dye marker (step 5) or autofluorescence signals may be recorded by the fluorescence imaging microscopy method.

The luminescent marker may be configured and designed to selectively label only microbes.

The luminescent marker may be a fluorescent dye or compound, configured to stain microbes, such as bacteria independently of their metabolic state. Such dyes are for example, known as SYTO dyes, DAPI, Sybr-Green.

Alternatively, the luminescent marker may be configured to specifically bind to bacteria of a certain kind. Such dye may be comprised by a compound comprising vancomycin.

The notion of labelling particularly comprises the notion of staining.

The notion of microbes particularly comprises the notion of bacteria, procaryotic cells, germs.

After the fluorescent marker labelling step 5, a washing step 6 ay be executed that washes away any unbound fluorescent marker before the optical imaging 7 is performed.

The obtained location of possible microbes may then guide the Raman-spectroscopic measurement (step 8), which may be implemented as a point-measurement.

The Raman illumination may be provided by a laser, that is focused onto the surface of the filter onto the location, where the microbes have been detected with the optical imaging method. In case several locations have been detected, the Raman laser may be positioned on the locations in a serial fashion such that for each location a Raman, spectrum is obtained.

The term "Raman spectrum" particularly comprises the notion of a portion of a Raman spectrum, i.e being limited only to a particularly predefined wavenumber interval.

The range of wavenumber for which the Raman-spectrum is recorded may be selected according to the expected Raman-signal of the Raman-isotope(s) used for incubation.

The Raman-signal, i.e. the Raman-spectrum carries information on whether the Raman-active isotope is present at the location.

In case the Raman-active isotope is present, it may be concluded 9 that the microbe at the location was active, i.e. alive, at the time of the incubation step. In case no Raman-active isotope signature can be found in the Raman-spectrum, it may be concluded 9 that the microbe at the location has been inactive or dead at the time of incubation.

From the signal strength and particularly also from the Raman-spectrum it may be concluded that a mixture of dead and alive microbes at the time of incubation are present at the location.

By classifying 10 the microbes and/or by estimating 11 the number of dead and alive microbes it is then possible to generate 12 an indicator reflecting a potential health hazard or contamination.

From the Raman-spectrum an identification, i.e. a classification 10 of the microbe may be performed, wherein a classifier may assign the recorded Raman-signal to a microbe exhibiting the same or a similar Raman-spectrum. For this purpose, a database comprising the Raman-spectra of a plurality of microbes may be accessed and a comparison of the recorded Raman-spectrum with the Raman-spectra in the database can be executed. Alternatively, a trained classifier such as an artificial neural network may assign the recorded Raman-spectrum to a kind or type of microbe.

The filter may be configured to provide particularly advantageous properties with regard to the acquisition of the Raman-spectrum, which are disclosed in the following.

The filter surface may consist of or comprise a reflective and smooth aluminium layer on which the microbes are retained. Such a surface provides a Raman-neutral background (particularly in contrast to polymer surfaces or polymer filters) that allows examination of individual bacteria. Further, an enhancement of the Raman signal by the mirror effect of the surface is achieved by the aluminium.

The thin aluminium layer, particularly in the order of 200 nm to 500 nm, may be arranged on top of a polymer carrier, that provides structural stability to the filter and reduces manufacturing costs of the filter.

## Claims

1. A method for identifying a metabolic state of microbes based on a Raman-spectroscopic signal, the method comprising the steps of:
a. Acquiring a sample comprising the microbes,
b. Exposing the sample to a solution comprising a Raman-active compound comprising a Raman-active isotope, wherein said compound is ingestible by the microbes by way of their metabolism,
c. Filtering the sample onto a filter that is designed and configured to retain the microbes on a surface of the filter and to clear the sample from the solution,
d. Imaging the filter surface with the microbes by means of an optical imaging method so as to obtain from the detected signal of the filter surface locations on the filter at which at least microbic residues or microbes are located,
e. On the filter, at least at one obtained location, performing a Raman-spectroscopic measurement configured to be sensitive to the Raman-active isotope and recording a Raman spectrum at the at least one obtained location,
f. From the recorded Raman spectrum recorded at the obtained location, differentiating between at least two metabolic states of the microbe at the time of exposure of the sample to the Raman active isotope by means of analysis of the Raman spectrum with regard to a corresponding signal in the Raman spectrum.

2. The method according to claim 1, wherein the optical imaging method comprises a label-free optical microscopy imaging method, particularly a dark-field and/or a differential interference contrast (DIC) microscopy method.

3. The method according to claim 1 or 2, wherein after the filtering step c, a labelling step is executed, wherein the labeling step comprises labeling the filtered sample on the surface with a Raman-spectroscopy-compatible luminescent marker that is configured to label microbes independent of their metabolic state, such that microbes on the filter surface may be labelled by the luminescent marker, and wherein the optical imaging method comprises a fluorescence imaging method configured to detect the luminescence of the luminescent marker.

4. The method according to claim 3, wherein the luminescent marker is a DNA-binding luminescent marker or an intercalating luminescent dye.

5. The method according to any of the claims 3 or 4, wherein the luminescent marker is attached to a microbe-specific marker, such as vancomycin or polymyxine, such that the luminescent marker is configured to only bind to gram-positive or only to gram-negative microbes.

6. The method according to any of the preceding claims, wherein after step b and prior to filtering the sample in step c and, the sample is treated with a denaturing agent, particularly a denaturing detergent, particularly wherein the agent comprises or consists of sodium dodecyl sulfate (SDS) or sodium lauryl sulfate (SLS).

7. The method according to any of the preceding claims, wherein the Raman-active compound is deuterated water.

8. The method according to any of the preceding claims, wherein a first metabolic state of the at least two metabolic states comprises or is an active metabolic state **characterized by** incorporation of the Raman-active isotope into metabolic products, such as proteins, RNA, DNA, lipids and/or organelles of the microbe, wherein a second metabolic state of the at least two metabolic states comprises or is a dead state **characterized in that** after the exposure step the microbe is devoid of the Raman-active isotope incorporated into metabolic products of the microbe.

9. The method according to any of the preceding claims, wherein the surface of the filter is a metallic and reflective surface.

10. The method according to any of the preceding claims, wherein the Raman spectroscopic measurement is recorded in the range of 2,100 cm⁻¹ to 2,400 cm¹.

11. The method according to any of the preceding claims, wherein from the Raman-spectrum a fraction of microbes in the first state and in the second state is determined, and wherein in case the faction is above or below a predefined threshold value, the method issues an alarm notice.

12. The method according to any of the preceding claims, wherein a determination of the metabolic state of the microbe is based on an analysis of only a selected spectral region of the Raman spectrum, particularly a spectral region that comprises information about a presence of the Raman-active isotope in the microbe.

13. The method according to any of the preceding claims, wherein from the recorded Raman spectrum, a kind or a type of the microbe is determined, particularly by means of a computer-based and computer-executed comparison to a database of Raman spectra exhibited by different types or kinds of microbes.

14. A kit comprising components for preparing a sample for identification of a metabolic state of microbes with the method according to any of the preceding claims, wherein the kit comprises a solution with a Raman-active compound, a luminescent dye configured to label microbes independently of a metabolic state, a filter having filter holes, wherein the filter is configured to retain microbes from an aqueous solution on a surface of the filter, wherein the surface of the filter is a metallic and reflective surface, such as to exhibit a diminished Raman-background signal.

15. The kit according to claim 14, wherein the surface of the filter is made of aluminium arranged on top of a polymer layer or wherein the surface is a metal foil, wherein the surface has a thickness between 200 nm to 400 nm, particularly between 210 nm and 250 nm.

## Amended claims

### Amended claims in accordance with Rule 137(2) EPC.

1. A method for identifying a metabolic state of microbes based on a Raman-spectroscopic signal, the method comprising the steps of:
a. Acquiring a sample comprising the microbes,
b. Exposing the sample to a solution comprising a Raman-active compound comprising a Raman-active isotope, wherein said compound is ingestible by the microbes by way of their metabolism,
c. Filtering the sample onto a filter that is designed and configured to retain the microbes on a surface of the filter and to clear the sample from the solution,
d. Imaging the filter surface with the microbes by means of an optical imaging method so as to obtain from the detected signal of the filter surface locations on the filter at which at least microbic residues or microbes are located,
e. On the filter, at least at one obtained location, performing a Raman-spectroscopic measurement configured to be sensitive to the Raman-active isotope and recording a Raman spectrum at the at least one obtained location,
f. From the recorded Raman spectrum recorded at the obtained location, differentiating between at least two metabolic states of the microbe at the time of exposure of the sample to the Raman active isotope by means of analysis of the Raman spectrum with regard to a corresponding signal in the Raman spectrum, wherein after step b. and prior to filtering the sample in step c., the sample is treated with a denaturing agent.

2. The method according to claim 1, wherein the optical imaging method comprises a label-free optical microscopy imaging method, particularly a dark-field and/or a differential interference contrast (DIC) microscopy method.

3. The method according to claim 1 or 2, wherein after the filtering step c, a labelling step is executed, wherein the labeling step comprises labeling the filtered sample on the surface with a Raman-spectroscopy-compatible luminescent marker that is configured to label microbes independent of their metabolic state, such that microbes on the filter surface may be labelled by the luminescent marker, and wherein the optical imaging method comprises a fluorescence imaging method configured to detect the luminescence of the luminescent marker.

4. The method according to claim 3, wherein the luminescent marker is a DNA-binding luminescent marker or an intercalating luminescent dye.

5. The method according to any of the claims 3 or 4, wherein the luminescent marker is attached to a microbe-specific marker, such as vancomycin or polymyxine, such that the luminescent marker is configured to only bind to gram-positive or only to gramnegative microbes.

6. The method according to any of the preceding claims, wherein the agent comprises or consists of sodium dodecyl sulfate (SDS) or sodium lauryl sulfate (SLS).

7. The method according to any of the preceding claims, wherein the Raman-active compound is deuterated water.

8. The method according to any of the preceding claims, wherein a first metabolic state of the at least two metabolic states comprises or is an active metabolic state **characterized by** incorporation of the Raman-active isotope into metabolic products, such as proteins, RNA, DNA, lipids and/or organelles of the microbe, wherein a second metabolic state of the at least two metabolic states comprises or is a dead state **characterized in that** after the exposure step the microbe is devoid of the Raman-active isotope incorporated into metabolic products of the microbe.

9. The method according to any of the preceding claims, wherein the surface of the filter is a metallic and reflective surface.

10. The method according to any of the preceding claims, wherein the Raman spectroscopic measurement is recorded in the range of 2,100 cm⁻¹ to 2,400 cm⁻¹.

11. The method according to any of the preceding claims, wherein from the Ramanspectrum a fraction of microbes in the first state and in the second state is determined, and wherein in case the faction is above or below a predefined threshold value, the method issues an alarm notice.

12. The method according to any of the preceding claims, wherein a determination of the metabolic state of the microbe is based on an analysis of only a selected spectral region of the Raman spectrum, particularly a spectral region that comprises information about a presence of the Raman-active isotope in the microbe.

13. The method according to any of the preceding claims, wherein from the recorded Raman spectrum, a kind or a type of the microbe is determined, particularly by means of a computer-based and computer-executed comparison to a database of Raman spectra exhibited by different types or kinds of microbes.

14. A kit comprising components for preparing a sample for identification of a metabolic state of microbes with the method according to claims 1 and 3, wherein the kit comprises a solution with the Raman-active compound comprising the Raman-active isotope, a luminescent dye configured to label microbes independently of a metabolic state, the filter having filter holes, wherein the filter is configured to retain microbes from an aqueous solution on a surface of the filter, wherein the surface of the filter is a metallic and reflective surface, such as to exhibit a diminished Raman-background signal.

15. The kit according to claim 14, wherein the surface of the filter is made of aluminium arranged on top of a polymer layer or wherein the surface is a metal foil, wherein the surface has a thickness between 210 nm and 250 nm.
